# EUROPEAN PATENT SPECIFICATION

(11) **EP 3 383 984 B1**
(45) Date of publication and mention of the grant of the patent: **11.03.2020**
(21) Application number: 15820687.0
(22) Date of filing: 04.12.2015
(51) Int. Cl.: C11D 1/00, C11D 3/34, C11D 3/37, C11D 9/22, C11D 9/32, C11D 17/00, C11D 3/22

(54) **CLEANSING BARS WITH TAURINE**
REINIGUNGSSTÜCKE MIT TAURIN
PAINS NETTOYANTS CONTENANT DE LA TAURINE

(43) Date of publication of application: 10.10.2018
(73) Proprietor: Colgate-Palmolive Company, New York, NY 10022 (US)
(72) Inventor: WANG, Jun, Fort Washington, Pennsylvania 19034 (US); DUBOVOY, Viktor, Cresskill, New Jersey 07626 (US); NABI, Zeenat, Cranbury, New Jersey 08512 (US); MASTRULL, Jeffrey, Piscataway, NJ 08854 (US); CHENG, Shujiang, Warren, New Jersey 07059 (US); DU-THUMM, Laurence, Princeton, New Jersey 08540 (US); PAN, Long, Somerset, New Jersey 08873 (US)
(74) Representative: Wichmann, Hendrik
(86) International application number: PCT/US2015/064065
(87) International publication number: WO 2017/095445

(56) References cited:
- WO-A1-2015/088489
- US-A- 5 786 312
- US-B1- 6 352 965
- DATABASE WPI Week 200174 Thomson Scientific, London, GB; AN 2001-641706 XP002760220, & JP 2001 200294 A (NIPPON OILS & FATS CO LTD) 24 July 2001 (2001-07-24)

## Description

### BACKGROUND

Taurine (2-aminoethanesulfonic acid) is a natural organic acid present in many animal organisms. Taurine is essential to the healthy maintenance of skeletal muscle, cardiovascular function, and central nervous system function. It is also an anti-oxidant and it helps stabilize cellular membranes.

Over the last 15 years, taurine and taurine derivatives have been used in a variety of cosmetic and personal care compositions, including hair conditioners, moisturizers, cleansing products, shaving cream and after-shave compositions. Taurine is suspected to have anti-fibrotic properties and has been shown to protect hair follicles from damage caused by the transforming growth factor family of proteins. Taurine also helps maintain skin hydration, contributing to the healthy maintenance of the skin barrier, and helps reduce inflammation and/or irritation of the skin.

Most of the cosmetic and personal care products that contain taurine are non-solid compositions, such as gels, creams, lotions, and liquids. In attempting to formulate a solid cleansing bar composition containing taurine, the inventors have found that under normal aging conditions (e.g., dry room temperature aging), gritty solid particles or crystals of taurine precipitate out of the composition on its surface. This gives the cleansing bar an unwelcome abrasive feel. Consequently, there is a need for formulating cleansing soap bars that deliver the beneficial effects of taurine without undesirable precipitation of taurine on the surface.

WO2015/088489 A1 discloses a method for reducing or inhibiting the crystallization of taurine in a soap bar. US6352965 discloses soap bars comprising tourate, surcrose and POP9 diglycerylether.

### SUMMARY

The inventors have discovered that taurine can be stabilized in a solid cleansing bar composition by the inclusion of a polymer. A cleansing bar composition and a method of inhibiting taurine crystallization in a cleansing bar according to the present invention are defined in the claims.

The present disclosure provides a cleansing bar composition comprising:
a) at least one cleanser chosen from soap and surfactant;
b) taurine, in free or salt form; and
c) at least one polymer,
   wherein the composition is a solid cleansing bar.

Also disclosed is a method of inhibiting taurine crystallization in a cleansing bar comprising
a) combining taurine, in free or salt form with a polymer to form a mixture;
b) adding the mixture to at least one cleanser chosen from soap and surfactant; and
c) forming a cleansing bar.

The present disclosure also provides for the use of a polymer to prevent the crystallization of taurine when taurine is incorporated into a bar soap.

### DETAILED DESCRIPTION

Unless otherwise specified, all percentages and amounts expressed herein and elsewhere in the specification should be understood to refer to percentages by weight. As used throughout, ranges are used as shorthand for describing each and every value that is within the range. Any value within the range can be selected as the terminus of the range.

As used herein, the term "cleansing bar" shall include bars for cleansing and personal hygienic use comprising a cleanser chosen from soap and surfactant. The cleansing bar may be a soap bar (soap is the cleanser), syndet (non-soap surfactant is the cleanser), or combar (a mixture of soap and surfactant).

The present disclosure provides cleansing bars containing one or more cleansing materials, taurine, and a polymer. The polymer is a polyethylene oxide-polypropylene oxide block copolymer.

In one exemplary embodiment, the present disclosure provides a cleansing bar composition (Composition 1) comprising:
a) at least one cleanser component chosen from soap and surfactant;
b) taurine, in free or salt form; and
c) at least one polymer.

The inventors have discovered that taurine can be stabilized in cleansing bars by the addition of the polymer. The polymer prevents the precipitation (e.g., crystallization) of taurine on the surface of the cleansing bar as it ages. Also, the polymer can increase the whiteness of the soap when taurine is included.

The polymer is a polyethylene oxide-polypropylene oxide block copolymer.

Polyethylene oxide-polypropylene oxide block copolymers are commonly known by the generic term "poloxamer", and have the general formula (EO)ₓ(PO)_{y}(EO)_{z}, with x, y and z being selected depending on the number average molecular weight of the PO portion and the percentage of EO in the copolymer. These copolymers are designated by a three digit number: the first two digits x 100 give the approximate molecular mass of the polyoxypropylene block, and the last digit x 10 gives the EO percentage. In one embodiment, the number average molecular weight of the polyoxypropylene block is 1500 to 15000, and the percentage of polyoxyethylene is 10 to 90%. In another embodiment, the number average molecular weight of the polyoxypropylene block is 2500 to 4500, and the percentage of polyoxyethylene is 10 to 90%. In another embodiment, the number average molecular weight of the polyoxypropylene block is 3000 to 4000, and the percentage of polyoxyethylene is 10 to 80%. In another embodiment, the number average molecular weight of the polyoxypropylene block is 3600 to 4000, and the percentage of polyoxyethylene is 70%.

In one embodiment, the polyethylene oxide-polypropylene oxide block copolymer is poloxamer 407 (which has a polyoxypropylene molecular mass of approximately 4,000 g/mol and a 70% polyoxyethylene content). In one embodiment, the polyethylene oxide-polypropylene oxide block copolymer can be purchased as Pluronic F127 from BASF. In this designation, the copolymers start with a letter that indicates the physical form at room temperature (L = liquid, P = paste, F = flake), which is then followed by two or three digits, The first digit (or two digits in a three-digit number) in the designation is multiplied by 300 to indicate the approximate molecular weight of the PO portion; and the last digit x 10 gives the EO percentage. Pluronic F127 thus has a molecular mass of approximately 3600 g/mol and a 70% EO content). In other embodiments, the polyethylene oxide-polypropylene oxide block copolymer is Pluronic F108 (MW of 3000 and 80% EO), Pluronic L101 (MW of 3000, 10% EO), Pluronic P123 (MW of 3600, 30% EO), or Pluronic P104 (MW of 3000, 40% EO).

In preparing the cleansing bars of the present disclosure, the taurine component can be pre-mixed with the polymer, e.g., encapsulated by the polymer, prior to blending with the other components of the cleansing bar composition. Alternatively, the taurine can blended into the other components of the cleansing bar composition, before or after the addition of the polymer. Preferably, the taurine and polymer are pre-mixed and processed to ensure coating of the surface area of the taurine particles by the polymer.

The cleansing bar of the present disclosure includes at least one cleanser component. In certain embodiments the cleanser component is a hydrophilic soap chip (*e.g*., "a base component"). The term "soap" or "soap chip" is used herein in its popular sense, *i.e*., the alkali metal or alkanol ammonium salts of aliphatic alkane or alkene monocarboxylic acids. Sodium, potassium, mono-, di- and tri-ethanol ammonium cations, or combinations thereof, are suitable for purposes of this invention. In general, sodium soaps are used in the compositions of the present disclosure, but from about 1% to about 25% of the soap may be ammonium, potassium, magnesium, calcium soaps or a mixture of these soaps.

The soap chips useful herein include, but are not limited to, the well-known alkali metal salts of aliphatic (alkanoic or alkenoic) acids having about 8 to 22 carbon atoms, preferably 10 to 20 carbon atoms. These may be described as alkali metal carboxylates of alkanoic or alkenoic hydrocarbons having about 12 to about 22 carbon atoms. Soaps having the fatty acid distribution of common vegetable oils may be suitable, e.g., palm kernel oil, palm oil, coconut oil, olive oil or laurel oil, or the fatty acid distribution of tallow (rendered animal fat). The soap may comprise the fatty acid distribution of any combination of natural or synthetic fatty acid sources (e.g., any combination of natural animal or vegetable fats or oils, and/or individual fatty acids).

Any other surfactant can also be present in the soap chip which include but are not limited to sulfate, sulfonate alpha olefin sulfonates, isethionates such as SCI, N-alkyl or N-acyl taurates, sulfosuccinate, phosphates, glycinates, amphoteric surfactants such as betaines, sulfobetaines and the like and nonionic surfactants such as alkanolamide, alkylpolyglycosides.

In one exemplary embodiment, the cleansing bar of this disclosure includes at least 70% by weight of cleanser active compounds (e.g., soap active compounds).

In an alternate exemplary embodiment, the cleanser of the composition consists essentially of anionic surfactant, non-ionic surfactants, amphoteric surfactants, cationic surfactants and mixtures thereof.

Optional ingredients can be present in the cleansing bar composition. Non-limiting examples include skin conditioning agents, moisturizing agents, fragrance, dyes and pigments, titanium dioxide, chelating agents such as EDTA, sunscreen active ingredients such as butyl methoxy benzoylmethane; antiaging compounds such as alpha hydroxy acids, beta hydroxy acids; antimicrobial materials such as triclocarban, triclosan and the like; preservatives such as hydantoins, imidazolines; polyols such as glycerol, sorbitol, propylene glycol and polyethylene glycols; particulate matter such as silica, talc, or calcium carbonate; antioxidants such as butylated hydroxytoluene (BHT); vitamins such as A, E, K and C; essential oils and extracts thereof such as rosewood and jojoba, particulate matter such as polyethylene beads, jojoba beads, lufa, or oat flour, and mixtures of any of the foregoing components.

In one embodiment the cleansing bar includes fragrance in an amount of 0.001% to 2% by weight of the composition.

In one embodiment the cleansing bar includes pearlizers, such as titanium dioxide, in an amount of 0.01% to 1% by weight.

In one embodiment the cleansing bar includes one or more pigments, such as chromium oxide green, in an amount of 0.001% to 1% by weight.

In one embodiment, the cleansing bar includes silica, or silicon dioxide, incorporated at a level of from about 0.1% to about 15%, preferable from about 1% to about 10%, more preferably from about 3% to about 7%. Silica is available in a variety of forms, including but not limited to, crystalline, amorphous, fumed, precipitated, gel, and colloidal forms.

In one embodiment, the cleansing bar includes free fatty acids to provide enhanced skin feel benefits, such as softer or smoother feeling skin. Suitable free fatty acids include those derived from tallow, coconut oil, palm oil and palm kernel oil.

In a second exemplary embodiment, the invention includes a method (Method 1) of inhibiting taurine precipitation (e.g., crystallization) on the surface of a cleansing bar, comprising
a) combining taurine, in free or salt form, with a polyethylene oxide-polypropylene oxide block copolymer to form a mixture;
b) adding the mixture to at least one cleanser chosen from soap and surfactant; and
c) forming a cleansing bar.

The cleansing bars of the present disclosure may be prepared by any of the techniques known to those skilled in the art, including both batch processes and continuous processes. The first step in the preparation of the cleansing bar is the preparation of the soap component. Techniques known to those skilled in the art may be used, such as the classic kettle boiling process or the modern continuous soap manufacturing process. For example, an appropriate fat, oil, or carboxylic acid, or mixture thereof, is first combined with a base (e.g, sodium or potassium hydroxide or carbonate) in the presence of water to form the soap component. The soap component can then be processed and purified to remove excess base and/or glycerol as needed, and formed into chips, pellets, noodles or other solid or semi-solid forms. Optional ingredients such as additional surfactants may also be added after the removal of excess base but before formation into chips, pellets or noodles. The soap component may then be ground up, suspended in water and combined with the taurine and polymer, as well as other optional additives. Preferably, the taurine and polymer are pre-mixed, with melting of the polyethylene oxide-polypropylene oxide block copolymer, if necessary. The mixture is processed, as by stirring or grinding to promote an even coating of the taurine particles by the polymer. The resulting mixture is then blended, with heating if necessary, with the soap chips and any other desired ingredients. After blending, the final composition is then formed into the finished cleansing bar product.

The cleansing bar may be formed by the extrusion method, and may be of varying sizes and shapes such as ovoid or rectangular in shape with either a flat or curved profile as an overall appearance.

Exemplary embodiments of the present disclosure will be illustrated by reference to the following examples, which are included to exemplify, but not to limit the scope of the present invention.

### EXAMPLES

### Example 1: Analysis of Stabilization Effect

2.50 grams of taurine crystals are placed into a mortar and ground up with a pestle. 2.50 g of polyethylene oxide-polypropylene oxide block copolymer is added. The mixture is thoroughly mixed and ground. Separately, a soap slurry is prepared by grinding up super-fat soap chips to a fine powder and mixing with water in a 1:0.3 ratio by weight. 60 g of this soap slurry is added to taurine/polyethylene oxide-polypropylene oxide block copolymer mixture and mixed to homogeneity. A control is also prepared without the copolymer in which water is added in its place. A small portion of the inventive and control are transferred to syringes and squeezed out to form a "noodle" on a microscope slide.

The noodles on the microscope slides are aged for two weeks at room temperature before being viewed with an Olympus SZX10 stereo microscope and photomicrographs of the noodles of the compositions are analyzed. During aging, most of the water in the soap composition evaporates, so that the final compositions are about 5 wt% taurine and about 5 wt% polymer. The control composition, containing water in place of a polyethylene oxide-polypropylene oxide block copolymer, has clear crystals on the surface of the noodles. In contrast, the composition containing polyethylene oxide-polypropylene oxide block copolymer has substantially reduced levels of crystallization.

### Example 2: Color Measurement

The preparation as in Example 1 is repeated. The soap noodles are aged in a 50°C oven for 7 days and color variance is quantified using a SpectroShade spectrophotometer to measure L, a, and b values, and W is calculated as W=(a2 + b2 + (L-100)2)1/2 as compared to unaged soap chips. The results are described in Table 1 below. A lower ΔW indicates less discoloration compared to the unaged control.

**Table 1**

| **Composition** | **W** | **ΔW** |
|---|---|---|
| Unaged Soap Chips | 10.8 | -- |
| Example I (taurine w/polymer) | 23.5 | 12.7 |
| Comparative Example (taurine w/o polymer) | 28.8 | 18.0 |

As illustrated by the data described in Table 1 (above), the addition of an exemplary polymer of the present invention, polyethylene oxide-polypropylene oxide block co-polymer, unexpectedly reduces discoloration in aged soap bars containing taurine.

## Claims

1. A cleansing bar composition comprising:
a) at least one cleanser chosen from soap and surfactant;
b) taurine, in free or salt form; and
c) at least one polymer comprising a polyethylene oxide-polypropylene oxide block copolymer;
wherein the composition is a solid cleansing bar, and
wherein the weight ratio of taurine to polymer is from 1:4 to 4:1.

2. The composition of claim 1, wherein the taurine is substantially coated by the polymer.

3. The composition of claim 1 or 2, wherein the taurine is in salt form and is selected from the group consisting of taurine hydrochloride, taurine sulfate, taurine acetate, alkali metal salt of taurine, alkaline earth metal salt of taurine, or ammonium salt of taurine.

4. The composition of any preceding claim, wherein the taurine is present in an amount of 0.1% to 10% by weight of the composition, 2% to 8% by weight of the composition,
4% to 6% by weight of the composition, or 5% by weight of the composition.

5. The composition of claim 1, wherein the polyethylene oxide-polypropylene oxide block copolymer has a number average molecular weight of the polyoxypropylene block of 1500 to 15000, and the percentage of polyoxyethylene is 10 to 90%, wherein the polyethylene oxide-polypropylene oxide block copolymer has a number average molecular weight of the polyoxypropylene block of 2500 to 4500, and the percentage of polyoxyethylene is 10 to 90%, wherein the polyethylene oxide-polypropylene oxide block copolymer has a number average molecular weight of the polyoxypropylene block of 3000 to 4000, and the percentage of polyoxyethylene is 10 to 80%, or wherein the polyethylene oxide-polypropylene oxide block copolymer has a number average molecular weight of the polyoxypropylene block of 3600 to 4000, and the percentage of polyoxyethylene is 70%.

6. The composition of any preceding claim, wherein the at least one polymer comprises 0.1% to 10% by weight of the composition, 2% to 8% by weight of the composition, 4% to 6% by weight of the composition, or 5% by weight of the composition.

7. The composition of any preceding claim, wherein the at least one polymer prevents crystallization of taurine on the surface of the cleansing bar.

8. The composition of any preceding claim, wherein the at least one cleanser comprises a soap, for example, an alkali metal or alkylammonium salt of a C8-C22 carboxylic acid.

9. A method of inhibiting taurine crystallization in a cleansing bar comprising
a) combining taurine, in free or salt form in a polymer comprising a polyethylene oxide-polypropylene oxide block copolymer to form a mixture, wherein the weight ratio of taurine to polymer is from 1:4 to 4:1;
b) adding the mixture to at least one cleanser chosen from soap and surfactant; and
c) forming a cleansing bar.

10. The method of claim 9, wherein the taurine is substantially coated by the polymer.

11. The method of any of claims 9-10, wherein the taurine is present in an amount of 0.1% to 10% by weight of the composition, 2% to 8% by weight of the composition, 4% to 6% by weight of the composition, or 5% by weight of the composition.

12. The method of any of claims 9-11, wherein the polyethylene oxide-polypropylene oxide block copolymer has a number average molecular weight of the polyoxypropylene block of 1500 to 15000, and the percentage of polyoxyethylene is 10 to 90%, wherein the polyethylene oxide-polypropylene oxide block copolymer has a number average molecular weight of the polyoxypropylene block of 2500 to 4500, and the percentage of polyoxyethylene is 10 to 90%, wherein the polyethylene oxide-polypropylene oxide block copolymer has a number average molecular weight of the polyoxypropylene block of 3000 to 4000, and the percentage of polyoxyethylene is 10 to 80%, or wherein the polyethylene oxide-polypropylene oxide block copolymer has a number average molecular weight of the polyoxypropylene block of 3600 to 4000, and the percentage of polyoxyethylene is 70%.

13. Use of a polymer comprising a polyethylene oxide-polypropylene oxide block copolymer to prevent crystallization of taurine and/or to reduce discoloration of a bar soap when taurine is incorporated into the bar soap, wherein the weight ratio of taurine to polymer is from 1:4 to 4:1.

## Patentansprüche

1. Reinigungsstückzusammensetzung, die umfasst:
a) zumindest ein Reinigungsmittel, das aus Seife und Tensid ausgewählt ist;
b) Taurin in freier oder in Salzform; und
c) zumindest ein Polymer, das ein Polyethylenoxid-Polypropylenoxid-Blockcopolymer umfasst;
wobei die Zusammensetzung ein festes Waschstück ist, und
wobei das Gewichtsverhältnis von Taurin zu Polymer 1:4 bis 4:1 beträgt.

2. Zusammensetzung nach Anspruch 1, wobei das Taurin im Wesentlichen durch das Polymer beschichtet ist.

3. Zusammensetzung nach Anspruch 1 oder 2, wobei das Taurin in Salzform vorliegt und aus der Gruppe ausgewählt ist, bestehend aus Taurinhydrochlorid, Taurinsulfat, Taurinacetat, Alkalimetallsalz von Taurin, Erdalkalimetallsalz von Taurin oder Ammoniumsalz von Taurin.

4. Zusammensetzung nach einem vorstehenden Anspruch, wobei das Taurin in einer Menge von 0,1 Gew.-% bis 10 Gew.-% der Zusammensetzung, 2 Gew.-% bis 8 Gew.-% der Zusammensetzung,
4 Gew.-% bis 6 Gew.-% der Zusammensetzung oder 5 Gew.-% der Zusammensetzung vorhanden ist.

5. Zusammensetzung nach Anspruch 1, wobei das Polyethylenoxid-Polypropylenoxid-Blockcopolymer ein zahlenmittleres Molekulargewicht des Polyoxypropylenblocks von 1500 bis 15000 aufweist und der Prozentsatz von Polyoxyethylen 10 bis 90 % beträgt, wobei das Polyethylenoxid-Polypropylenoxid-Blockcopolymer ein zahlenmittleres Molekulargewicht des Polyoxypropylenblocks von 2500 bis 4500 aufweist und der Prozentsatz von Polyoxyethylen 10 bis 90 % beträgt, wobei das Polyethylenoxid-Polypropylenoxid-Blockcopolymer ein zahlenmittleres Molekulargewicht des Polyoxypropylenblocks von 3000 bis 4000 aufweist und der Prozentsatz von Polyoxyethylen 10 bis 80 % beträgt oder wobei das Polyethylenoxid-Polypropylenoxid-Blockcopolymer ein zahlenmittleres Molekulargewicht des Polyoxypropylenblocks von 3600 bis 4000 aufweist und der Prozentsatz von Polyoxyethylen 70 % beträgt.

6. Zusammensetzung nach einem vorstehenden Anspruch, wobei das zumindest eine Polymer 0,1 Gew.-% bis 10 Gew.-% der Zusammensetzung, 2 Gew.-% bis 8 Gew.-% der Zusammensetzung, 4 Gew.-% bis 6 Gew.-% der Zusammensetzung oder 5 Gew.-% der Zusammensetzung umfasst.

7. Zusammensetzung nach einem vorstehenden Anspruch, wobei das zumindest eine Polymer eine Kristallisation von Taurin auf der Oberfläche des Reinigungsstücks verhindert.

8. Zusammensetzung nach einem vorstehenden Anspruch, wobei das zumindest eine Reinigungsmittel eine Seife, z. B. ein Alkalimetall- oder Alkylammoniumsalz einer C8-C22-Carbonsäure, umfasst.

9. Verfahren zum Hemmen der Kristallisation von Taurin in einem Reinigungsstück, das umfasst:
a) Kombinieren von Taurin in freier oder in Salzform in einem Polymer, das ein Polyethylenoxid-Polypropylenoxid-Blockcopolymer umfasst, um eine Mischung zu bilden, wobei das Gewichtsverhältnis von Taurin zu Polymer 1:4 bis 4:1 beträgt;
b) Zugeben der Mischung zu zumindest einem Reinigungsmittel, das aus Seife und Tensid ausgewählt ist; und
c) Bilden eines Reinigungsstücks.

10. Verfahren nach Anspruch 9, wobei das Taurin im Wesentlichen durch das Polymer beschichtet ist.

11. Verfahren nach einem der Ansprüche 9 bis 10, wobei das Taurin in einer Menge von 0,1 Gew.-% bis 10 Gew.-% der Zusammensetzung, 2 Gew.-% bis 8 Gew.-% der Zusammensetzung, 4 Gew.-% bis 6 Gew.-% der Zusammensetzung oder 5 Gew.-% der Zusammensetzung vorhanden ist.

12. Verfahren nach einem der Ansprüche 9 bis 11, wobei das Polyethylenoxid-Polypropylenoxid-Blockcopolymer ein zahlenmittleres Molekulargewicht des Polyoxypropylenblocks von 1500 bis 15000 aufweist und der Prozentsatz von Polyoxyethylen 10 bis 90 % beträgt, wobei das Polyethylenoxid-Polypropylenoxid-Blockcopolymer ein zahlenmittleres Molekulargewicht des Polyoxypropylenblocks von 2500 bis 4500 aufweist und der Prozentsatz von Polyoxyethylen 10 bis 90 % beträgt, wobei das Polyethylenoxid-Polypropylenoxid-Blockcopolymer ein zahlenmittleres Molekulargewicht des Polyoxypropylenblocks von 3000 bis 4000 aufweist und der Prozentsatz von Polyoxyethylen 10 bis 80 % beträgt oder wobei das Polyethylenoxid-Polypropylenoxid-Blockcopolymer ein zahlenmittleres Molekulargewicht des Polyoxypropylenblocks von 3600 bis 4000 aufweist und der Prozentsatz von Polyoxyethylen 70 % beträgt.

13. Verwendung eines Polymers, das ein Polyethylenoxid-Polypropylenoxid-Blockcopolymer umfasst, um die Kristallisation von Taurin zu verhindern und/oder eine Verfärbung einer Stückseife zu verringern, wenn Taurin in die Stückseife eingebunden ist, wobei das Gewichtsverhältnis von Taurin zu Polymer 1:4 bis 4:1 beträgt.

## Revendications

1. Composition de pain nettoyant comprenant :
a) au moins un nettoyant choisi parmi un savon et un tensio-actif ;
b) de la taurine, sous forme libre ou de sel ; et
c) au moins un polymère comprenant un copolymère à blocs poly(oxyde d'éthylène) - poly(oxyde de propylène) ;
dans laquelle la composition est un pain nettoyant solide, et
dans laquelle le rapport en poids de la taurine au polymère est de 1:4 à 4:1.

2. Composition selon la revendication 1, dans laquelle la taurine est enrobée de façon substantielle par le polymère.

3. Composition selon l'une des revendications 1 ou 2, dans laquelle la taurine est sous forme de sel et est choisie dans le groupe consistant en le chlorhydrate de taurine, le sulfate de taurine, l'acétate de taurine, un sel de métal alcalin de taurine, un sel de métal alcalino-terreux de taurine ou un sel d'ammonium de taurine.

4. Composition selon l'une quelconque des revendications précédentes, dans laquelle la taurine est présente dans une quantité de 0,1 % à 10 % en poids de la composition, de 2 % à 8 % en poids de la composition, de 4 % à 6 % en poids de la composition ou de 5 % en poids de la composition.

5. Composition selon la revendication 1, dans laquelle le copolymère à blocs poly(oxyde d'éthylène) - poly(oxyde de propylène) a un masse moléculaire moyenne en nombre du bloc polyoxypropylène de 1500 à 15000, et le pourcentage de polyoxyéthylène est de 10 à 90 %, dans laquelle le copolymère à blocs poly(oxyde d'éthylène) - poly(oxyde de propylène) a une masse moléculaire moyenne en nombre du bloc polyoxypropylène de 2500 à 4500, et le pourcentage de polyoxyéthylène est de 10 à 90 %, dans laquelle le copolymère à blocs poly(oxyde d'éthylène) - poly(oxyde de propylène) a une masse moléculaire moyenne en nombre du bloc polyoxypropylène de 3000 à 4000, et le pourcentage de polyoxyéthylène est de 10 à 80 %, ou dans laquelle le copolymère à blocs poly(oxyde d'éthylène) - poly(oxyde de propylène) a une masse moléculaire moyenne en nombre du bloc polyoxypropylène de 3600 à 4000, et le pourcentage de polyoxyéthylène est de 70 %.

6. Composition selon l'une quelconque des revendications précédentes, dans laquelle ledit au moins un polymère comprend 0,1 % à 10 % en poids de la composition, 2 % à 8 % en poids de la composition, 4 % à 6 % en poids de la composition ou 5 % en poids de la composition.

7. Composition selon l'une quelconque des revendications précédentes, dans laquelle ledit au moins un polymère empêche une cristallisation de taurine sur la surface du pain nettoyant.

8. Composition selon l'une quelconque des revendications précédentes, dans laquelle ledit au moins un nettoyant comprend un savon, par exemple, un sel de métal alcalin ou d'alkylammonium d'un acide carboxylique en C8-C22.

9. Procédé d'inhibition d'une cristallisation de taurine dans un pain nettoyant, comprenant :
a) combiner de la taurine, sous forme libre ou de sel, dans un polymère comprenant un copolymère à blocs poly(oxyde d'éthylène) - poly(oxyde de propylène) pour former un mélange, le rapport en poids de la taurine au polymère étant de 1 : 4 à 4 : 1 ;
b) ajouter le mélange à au moins un nettoyant choisi parmi un savon et un tensio-actif ; et
c) former un pain nettoyant.

10. Procédé selon la revendication 9, dans lequel la taurine est enrobée de façon substantielle par le polymère.

11. Procédé selon l'une quelconque des revendications 9 à 10, dans lequel la taurine est présente dans une quantité de 0,1 % à 10 % en poids de la composition, de 2 % à 8 % en poids de la composition, de 4 % à 6 % en poids de la composition ou de 5 % en poids de la composition.

12. Procédé selon l'une quelconque des revendications 9 à 11, dans lequel le copolymère à blocs poly(oxyde d'éthylène) - poly(oxyde de propylène) a une masse moléculaire moyenne en nombre du bloc polyoxypropylène de 1500 à 15000, et le pourcentage de polyoxyéthylène est de 10 à 90 %, dans lequel le copolymère à blocs poly(oxyde d'éthylène) - poly(oxyde de propylène) a une masse moléculaire moyenne en nombre du bloc polyoxypropylène de 2500 à 4500, et le pourcentage de polyoxyéthylène est de 10 à 90 %, dans lequel le copolymère à blocs poly(oxyde d'éthylène) - poly(oxyde de propylène) a une masse moléculaire moyenne en nombre du bloc polyoxypropylène de 3000 à 4000, et le pourcentage de polyoxyéthylène est de 10 à 80 %, ou dans lequel le copolymère à blocs poly(oxyde d'éthylène) - poly(oxyde de propylène) a une masse moléculaire moyenne en nombre du bloc polyoxypropylène de 3600 à 4000, et le pourcentage de polyoxyéthylène est de 70 %.

13. Utilisation d'un polymère comprenant un copolymère à blocs poly(oxyde d'éthylène) - poly(oxyde de propylène) pour empêcher une cristallisation de taurine et / ou pour réduire une altération de la couleur d'un pain de savon lorsque de la taurine est incorporée dans le pain de savon, le rapport en poids de la taurine au polymère étant de 1 : 4 à 4 : 1.
